# EUROPEAN PATENT SPECIFICATION

(11) **EP 4 403 639 B1**
(45) Date of publication and mention of the grant of the patent: **04.03.2026**
(21) Application number: 22786378.4
(22) Date of filing: 01.09.2022
(51) Int. Cl.: C05F 17/00

(54) **METHOD FOR THE PRODUCTION OF POLYHYDROXYALKANOATES AND ROOT BIOSTIMULANTS FROM ORGANIC WASTES**
VERFAHREN ZUR HERSTELLUNG VON POLYHYDROXYALKANOATEN UND WURZELBIOSTIMULANZIEN AUS ORGANISCHEN ABFÄLLEN
MÉTHODE POUR LA PRODUCTION DE POLYHYDROXYALCANOATES ET DE BIOSTIMULANT RACINAIRE À PARTIR DE DÉCHETS ORGANIQUES

(30) Priority: 14.09.2021 ES 202130854
(43) Date of publication of application: 24.07.2024
(73) Proprietor: QUÍMICA TÉCNICA ECOLÓGICA, S.L.U., 08221 Terrassa (Barcelona) (ES); Beda Water Engineering, S.L., 08860 Castelldefels (ES)
(72) Inventor: FERNÁNDEZ GARCÍA, Gustavo, 08221 Terrassa, Barcelona (ES); VALERO HERNÁNDEZ, Antonio, 08221 Terrassa, Barcelona (ES); MELERO TALAVERA, Sergio, 08221 Terrassa, Barcelona (ES)
(74) Representative: Isern Patentes y Marcas S.L.
(86) International application number: PCT/ES2022/070554
(87) International publication number: WO 2023/041822

(56) References cited:
- WO-A1-2019/119157
- WO-A1-2020/252582
- DE-B3- 102011 120 888
- MALHOTRA MILAN ET AL: "Biorefinery of anaerobic digestate in a circular bioeconomy: Opportunities, challenges and perspectives", RENEWABLE AND SUSTAINABLE ENERGY REVIEWS, vol. 166, 1 September 2022 (2022-09-01), US, pages 112642, XP093002201, ISSN: 1364-0321, DOI: 10.1016/j.rser.2022.112642
- SINGH RISHAN: "A Critical Review and Opinion on Hydrodistillation", BIO-SCIENCE RESEARCH BULLETIN, vol. 33, no. 1, 1 January 2017 (2017-01-01), pages 1, XP093336094, ISSN: 0970-0889, DOI: 10.5958/2320-3161.2017.00001.3
- A. T. WILLIAMS ET AL: "Ion exchange-precipitation for nutrient recovery from dilute wastewater", ENVIRON. SCI.: WATER RES. TECHNOL., vol. 1, no. 6, 1 January 2015 (2015-01-01), pages 832 - 838, XP055388626, ISSN: 2053-1400, DOI: 10.1039/C5EW00142K
- AZIZ ZARITH ASYIKIN ET AL: "Essential Oils: Extraction Techniques, Pharmaceutical And Therapeutic Potential - A Review", CURRENT DRUG METABOLISM, vol. 19, no. 13, 23 November 2018 (2018-11-23), US, pages 1100 - 1110, XP093336097, ISSN: 1389-2002, DOI: 10.2174/1389200219666180723144850
- MORETTO GIULIA ET AL: "An urban biorefinery for food waste and biological sludge conversion into polyhydroxyalkanoates and biogas", WATER RESEARCH, ELSEVIER, AMSTERDAM, NL, vol. 170, 5 December 2019 (2019-12-05), XP085983643, ISSN: 0043-1354, [retrieved on 20191205], DOI: 10.1016/J.WATRES.2019.115371
- ALLEGUE LUIS D ET AL: "Novel approach for the treatment of the organic fraction of municipal solid waste: Coupling thermal hydrolysis with anaerobic digestion and photo-fermentation", SCIENCE OF THE TOTAL ENVIRONMENT, ELSEVIER, AMSTERDAM, NL, vol. 714, 22 January 2020 (2020-01-22), XP086063134, ISSN: 0048-9697, [retrieved on 20200122], DOI: 10.1016/J.SCITOTENV.2020.136845
- PAPA GABRIELLA ET AL: "Implementing polyhydroxyalkanoates production to anaerobic digestion of organic fraction of municipal solid waste to diversify products and increase total energy recovery", BIORESOURCE TECHNOLOGY, vol. 318, 1 December 2020 (2020-12-01), AMSTERDAM, NL, pages 124270, XP093002189, ISSN: 0960-8524, DOI: 10.1016/j.biortech.2020.124270
- IVANOV V ET AL: "Production and applications of crude polyhydroxyalkanoate-containing bioplastic from the organic fraction of municipal solid waste", INTERNATIONAL JOURNAL OF ENVIRONMENTAL SCIENCE AND TECHNOLOGY, CENTER FOR ENVIRONMENT AND ENERGY RESEARCH AND STUDIES (C E E R S), IR, vol. 12, no. 2, 4 February 2014 (2014-02-04), pages 725 - 738, XP035423156, ISSN: 1735-1472, [retrieved on 20140204], DOI: 10.1007/S13762-014-0505-3
- RONGA DOMENICO ET AL: "Effects of solid and liquid digestate for hydroponic baby leaf lettuce (Lactuca sativa L.) cultivation", SCIENTIA HORTICULTURAE, vol. 244, 1 January 2019 (2019-01-01), AMSTERDAM, NL, pages 172 - 181, XP093003254, ISSN: 0304-4238, DOI: 10.1016/j.scienta.2018.09.037
- REN AI-TIAN ET AL: "Nutrient recovery from anaerobic digestion of food waste: impacts of digestate on plant growth and rhizosphere bacterial community composition and potential function in ryegrass", BIOLOGY AND FERTILITY OF SOILS, SPRINGER BERLIN HEIDELBERG, BERLIN/HEIDELBERG, vol. 56, no. 7, 26 May 2020 (2020-05-26), pages 973 - 989, XP037242935, ISSN: 0178-2762, [retrieved on 20200526], DOI: 10.1007/S00374-020-01477-6

## Description

### Object of the Invention

According to the title of this specification, the present invention relates to a process for the transformation of residual organic matter into PHA (polyhydroxyalkanoates) and compostable material for agriculture in accordance with the claims.

According to the process described herein, the residual organic matter generated by human activity will be used as raw material for the process described herein to generate, biodegradable plastic in the form of PHA and compostable material for agriculture, rich in organic matter and nitrogen, potassium and phosphorus.

### Background of the invention

Today, human activity generates a large amount of organic waste that is sent directly to the USW (Urban Solid Waste) landfill or is used in the production of biogas in anaerobic digesters or in agricultural composting.

There are processes which claim the production of PHA from organic waste originating from human activity, such as patents belonging to Genecis Bioindustries Inc., CA3086252, US019360008 or WO2020/252582, in which the way for producing PHA from organic waste, as well as the methodology to isolate the generated PHA are described. Patent ES 2397730 describes the methodology for manufacturing PHA from citrus waste and patent ES 2061405 specifies which type of bacteria is the most prolific in the production of PHA.

In no case there is described the way to generate and separate the compostable fraction for agriculture and the fraction that will go to the manufacture of PHA.

### Description of the invention

Therefore, the object of present invention is to provide a process and the reagents required for transforming organic waste generated by human activity (solid and liquid organic waste from supermarkets, hotels and bars, solid fraction from brown containers, organic livestock waste, organic waste from agricultural activity (fruit and pruning) and from the agricultural transformation industry (orange peel, avocado skin, etc.), sewage sludge, whey, etc.) into PHA and compostable material for agriculture, in accordance with the claims.

The first step in this process is the reception of the raw materials (previously called waste) for their subsequent transformation into PHA and compostable material.

The first step consists of separating the raw materials according to their origin, such that solid raw materials will be received and stored separately from liquid raw materials.

Different raw materials will be separated into 4 fractions according to their composition:
1.: Solid organic raw materials originating from supermarkets, hotels and restaurants, together with the organic matter from brown containers (organic fraction in selective collection), waste from pruning and from the agricultural and agri-food industry.
2.: Liquid organic raw materials: whey, milk and dairy products, sugary and alcoholic beverages, cleaning water from industrial processes for the production of musts, juices, etc.
3.: Sludge from WWTP and the output of anaerobic digesters.
4.: Livestock waste.

Based on the preceding 4 fractions, transformation is then performed in PHASES:

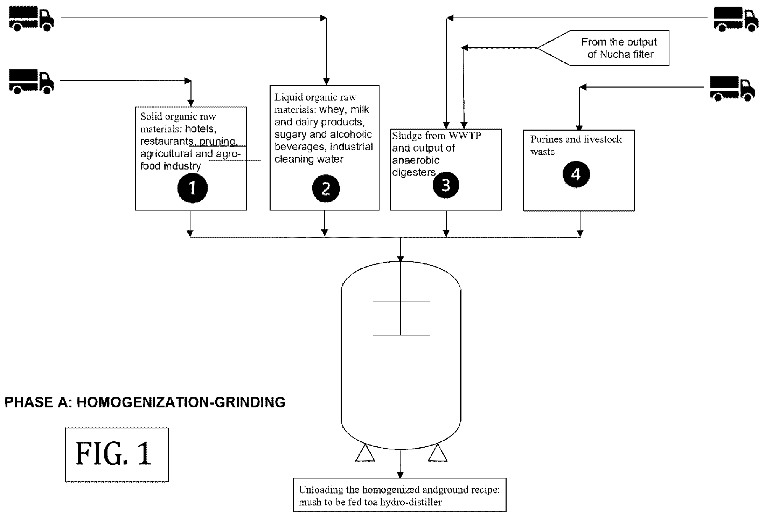

### PHASE A: HOMOGENIZATION-LIQUEFACTION: Obtaining mush for feeding a hydro-distiller. Figure 1

Before homogenizing each of the fractions, a recipe will be prepared, in which the mixture of each of the fractions (1 to 4) received will be indicated. Having established the recipe which indicates the mixture to be made from each of fractions 1 to 4, all solid and liquid raw materials will be mixed and homogenized in a reactor, in which the raw materials of the recipe will be homogenized-ground, obtaining an intimate mixture. The grinding of the mixture will generate a mush which, depending on the contribution of each fraction to the recipe, will have a variable concentration of solids having a size of less than 100 µm. The preparation of the recipe and its subsequent homogenization-grinding corresponds to PHASE A.

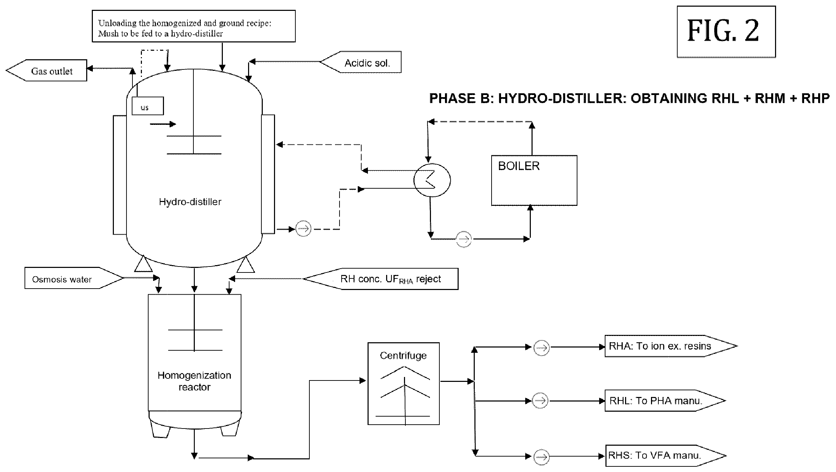

### Phase B: HYDRO-DISTILLER: OBTAINING RHL + RHM + RHP. Figure 2

The mixture obtained in phase A will feed a heated reactor with an outer jacket, or an inner jacket, or both, and an inner stirrer, having a reflux mechanism and an ultrasound system, in which and by means of stirring, the addition of chemical reagents and the supply of heat by means of the heated (inner and/or outer) jacket and ultrasound, as well as the recirculation of the steam generated and condensed during reflux, transformation will take place;
- Fats and oils will be transformed into fatty acids
- Glycogen and starch will be transformed into glucose monomers, fructose monomers, dextrins, etc.
- Proteins will be hydrolyzed into their basic components; amino acids (Aa) and small peptide chains.
- Solubilization of insoluble sulfates, carbonates and phosphates.

Proteins, sugars and fats from the mixture obtained in phase A, will be hydrolyzed into their monomers, by the action of heat + ultrasound + stirring + chemical reagents + reflux, inside the hydro-distiller (heated reactor), in addition to the sterilization-sanitization of the organic matter obtained in phase A. The alkaline earth phosphates, carbonates and sulfates will be solubilized by the addition of inorganic and/or organic acids, or mixtures thereof, together with adjuvants.

The residence time of each mixture originating from phase A inside the hydro-distiller, as well as the addition of chemical reagents (mixtures of organic and inorganic acids + adjuvants), will depend on the mixture of phase A itself.

Chemical reagents are added to facilitate protein, fat and sugar hydrolysis, as well as to dissolve the inorganic salts present in the mixture itself; to solubilized hydroxyapatite and calcium and magnesium carbonates originating from animal bone waste and mollusk shells, among others.

The chemical reagents used in the mixtures range from inorganic acids such as H₂SO₄, HCl, HNO₃, H₃PO₄, or mixtures thereof, together with organic acids such as lactic acids, glycolic acid, sulfamic acid, or mixtures thereof, including acid phosphonates or salts thereof, such as HEDP, ATMP, EDTMP, DTPMP, HDTMP, HEMPA or PBCT, acrylic acid homopolymers, acrylic and maleic acid polymers, terpolymers, etc.

The final composition of the mixture of acids and adjuvants will depend on the composition of the mixture of phase A and will be intended for inhibiting the precipitation of insoluble calcium and magnesium salts, as well as for hydrolyzing proteins, fats and carbohydrates into their basic components.

The hydro-distiller works discontinuously in batches. The phase A recipe is introduced together with the acid mixture into same, starting the hydrolysis-sterilization process. This process starts with the stirring of the mixture until the pH value on the inside is regulated to a value of 1 ≤ pH ≤ 6, depending on the recipe, together with the start of the recirculation of thermal oil through the inner/outer jacket of the hydro-distiller. Once the mixture inside the hydro-distiller has reached the pH value, the recirculation of the thermal oil through the outer/inner jacket of the reactor is maintained until reaching the working temperature and pressure therein. Once the setpoint T (temperature) and Pr. (pressure) value has been reached inside the reactor, and with stirring in progress, the steam generated inside the reactor, in the steam chamber of the reactor, is collected in a distillation column in which, and by means of external recirculation of water (or liquid from the next recipe to be introduced) at low temperature, the steam condenses and is reintroduced into the hydro-distiller by a steam reflux process. The time that the recipe is kept under stirring-reflux inside the hydro-distiller, the pH value, stirring time, the working temperature and pressure inside the hydro-distiller, are variables that depend on the Phase A recipe, for the purpose of sterilizing the mixture and releasing the liquid fraction, sugar monomers, amino acids and fatty acids. The working pH value inside the hydro-distiller ranges from 1 ≤ pH ≤ 6. The time at which the recipe is usually kept under stirring-heating, after reaching the desired pH value, ranges from 30 min to 120 min. The working pressures inside the hydro-distiller range from 2 bar to 5 bar and the working temperature ranges from 70°C to 110°C.

The hydro-distiller has, in addition to an stirrer, a US system that allows cell lysis and disintegration of organic matter. As such, stirring, heat input, the steam reflux system and the US allow obtaining monomers of sugars, fatty acids together with Aa + polypeptide chains in the aqueous acid solution inside the reactor.

After the stirring-reflux time of the Phase A recipe inside the hydro-distiller, the steam outlet valve (reflux) is closed, heat input is maintained for 1 min to 15 min, whereby the pressure inside the reactor rises to the desired value, at which time the hydro-distiller is depressurized by opening the steam outlet valve, collecting the fraction of the output steam on a steam trap with osmosis water.

After depressurizing the hydro-distiller, the contents of the reactor are emptied into a reception tank, homogenization reactor (HR), to which the osmosis water + condensates from the steam trap are added.

Work is performed with a 1:1 mixture of phase A recipe - osmosis water, where it is possible to reach up to 1:10, preferably 1:3.

The mixture of phase A recipe, output of the hydro-distiller + osmosis water in a ratio of 1:1 to 1:10, is homogenized inside the homogenization reactor by means of stirring. The pH is corrected if necessary and after 5 - 10 minutes of stirring-homogenization, the mixture from the homogenization reactor is fed to a centrifuge (Tricanter) which separates three fractions:
- Oils: The least dense fraction - RHL fraction: Light fraction
- Aqueous phase: The intermediate phase - RHA fraction: Aqueous Fraction
- Solids: the densest phase - RHS fraction: Solid fraction

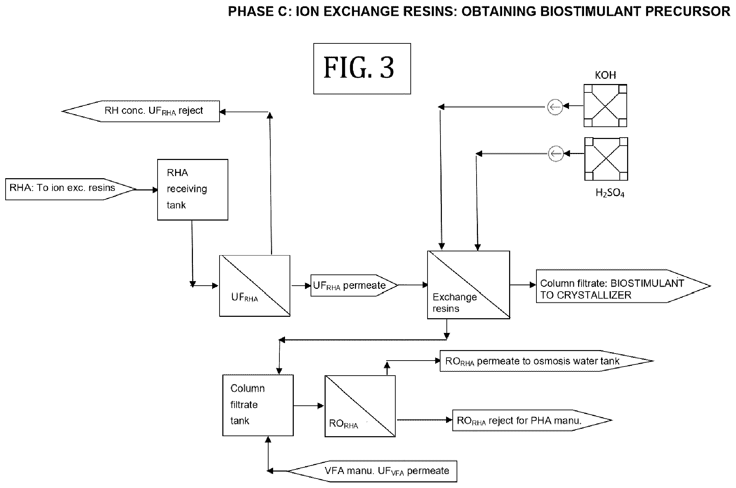

### PHASE C: ION EXCHANGE RESINS: OBTAINING BIOSTIMULATING PRECURSOR. Figure 3

Of the three phases obtained in the separation performed in the tricanter of Phase B, the RHA fraction is selected and filtered with the help of UF membranes with a molecular weight cut-off of 100,000 Daltons, UF_{RHA}.

The UF_{RHA} process generates two streams:
- A permeate stream: the fraction which has passed through the membranes and corresponds to 90-95% of the volume entering the plant
- A reject stream: the fraction which has not passed through the membrane. This fraction is recirculated over HR to the Tricanter-feeding tank.

The permeate fraction will feed a ion exchange resin system, formed by weak anionic resin + strong anionic resin - weak cationic resin + strong cationic resin. When the permeate from UF_{RHA} is passed through the ion exchange resins, the Aa released in the hydro-distiller will be retained in the resins together with the organic and inorganic salts present in the culture broth.

The filtered effluent will then be a sterile liquid solution which is rich in sugars (glucose, fructose, lactose, etc.) and has a very low conductivity.

The ion exchange columns, as a physical separation system, must be regenerated every time they become saturated. To that end, an acid and a base will be used for the regeneration thereof. A solution of inorganic acids, such as HCl, H₂SO₄ HNO₃, H₃PO₄ or mixtures thereof, will be used as acid, whereas KOH, NaOH, NH₃ or mixtures thereof will be used as bases.

In this case, KOH and H₂SO₄.will be used.

The regeneration solution will be an aqueous liquid solution rich in Aa + inorganic potassium salts and sulfates, which will be sent to an aerosol emission crystallizer to generate a solid biostimulant, rich in nitrogen (provided by the amino acids themselves), potassium and phosphorus. These last two cations come from KOH used as a regenerator in the case of anionic resins and from K⁺naturally occurring in fruits and vegetables, whereas phosphorus is supplied to the hydro-distiller by the hydroxyapatite of the animal and fish fraction.

Thus, the regeneration of the ion exchange resins successfully separates the nitrogen, phosphorus and potassium together with Aa, making up the root biostimulant which will be obtained in the form of dry powder at the outlet of the aerosol emission crystallizer.

The fraction filtered through the ion exchange columns, i.e., the eluate, is a fraction that is rich in sugars and has a low conductivity. This liquid fraction will be the fraction that will feed the aerated PHA-manufacturing reactor, together with the RHL fraction obtained in the tricanter.

PHA production by PHA-overproducing bacterial strains is much higher if they are fed directly with glucose than with starch or other sugar-rich raw materials. ATP manufacturing.

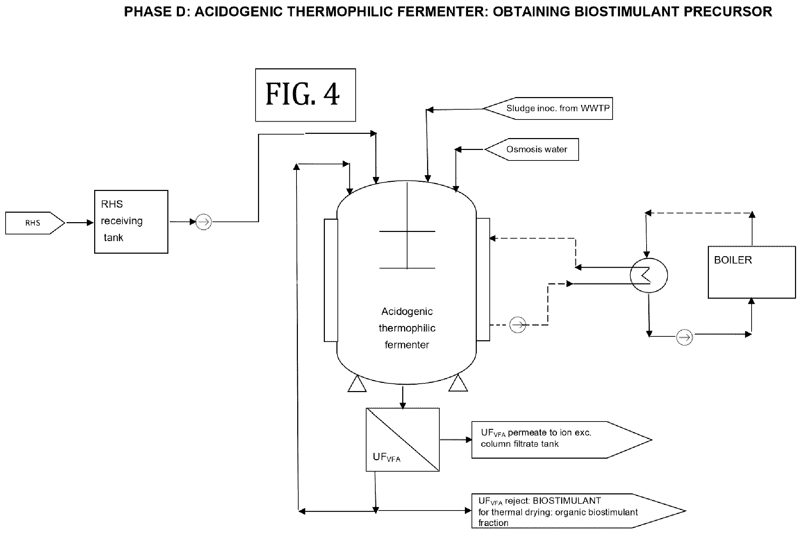

### PHASE D: ANAEROBIC BIOREACTOR - ACIDOGENIC THERMOPHILIC FERMENTER: OBTAINING VFA: Figure 4

The RHS fraction obtained through the tricanter at the outlet and discharge of the hydro-distiller will feed a reactor, i.e., an acidogenic thermophilic fermenter, in which, and under anaerobic conditions, VFA (volatile fatty acids) will be obtained from the RHS fraction.

The anaerobic reactor will be inoculated with activated sludge from WWTP (wastewater treatment plant), such that, and with the organic matter input from the RHS fraction, there will be generated, by means of anaerobic acidogenic fermentation, VFA (volatile fatty acids), made up mainly of acetic acid, propionic acid, butyric acid, isobutyric acid and isovaleric acid.

The acidogenic thermophilic fermenter will work in conditions of 5.5≤pH≤6.5 and at 35≤T°≤50, with a hydraulic retention time of 2 days.

VFA will be generated inside the acidogenic thermophilic fermenter from the continuously supplied organic matter (RHS fraction). The extraction of the VFA generated by the bacterial strains will be done by means of a multitubular UF_{VFA} plant that allows working up to 25% v/v of solids. By means of controlling the TSS, as well as the VFA/COD/T°/pH, using probes inside the acidogenic thermophilic fermenter, the continuous production of the biomass, as well as VFA, will be monitored.

The continuous UF_{VFA} plant will filter the contents of the reactor, generating two streams, a permeate stream which is rich in VFA and free of bacteria and a second fraction of solids at a maximum concentration of 25% v/v, which corresponds to the reject. This reject will be sent back to the thermophilic acidogenic fermenter again, while at the same time that the volume of permeate is replenished with more RHS fraction and osmosis water, until the TSS (total solid) concentration inside the reactor exceeds 25 g/l. At this point, the reject fraction is sent for thermal drying in order to obtain part of the organic fraction of the root biostimulant. This process is maintained until the value of the solids inside the reactor drops below 12 g/l, at which time what is purged from the UF_{VFA} is sent back to the acidogenic thermophilic fermenter again.

The permeate obtained by the UF_{VFA} membranes will be pH corrected to values of 7 ≤ pH ≤ 12 and will be treated in an RO_{RHA} plant. Two streams will be obtained from this plant:
- Permeate: Low conductivity osmosis water that will be sent back to the anaerobic VFA-manufacturing reactor
- Reject: Liquid VFA stream in the form salts + concentrated inorganic salts, that will be used to feed the PHA-manufacturing reactor, i.e., the RAPHA.

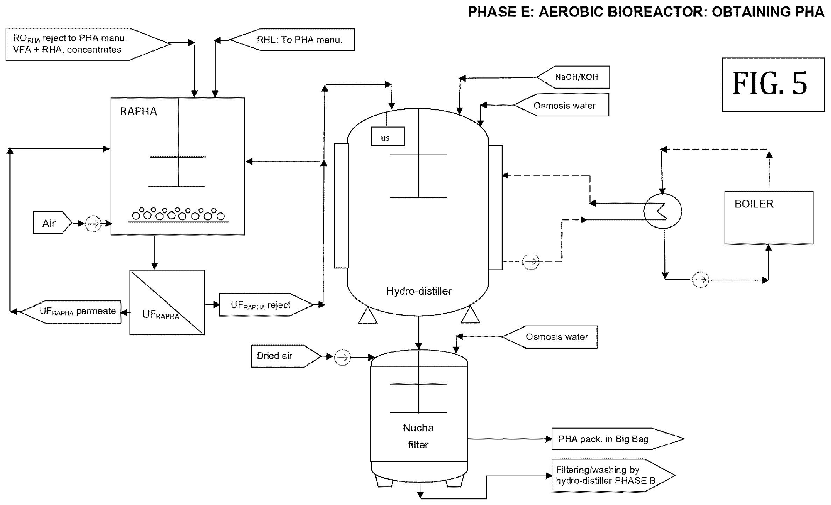

### PHASE E: AEROBIC BIOREACTOR: OBTAINING PHA. Figure 5.

The sugar fraction from the output of the ion exchange columns bound with the VFA-rich permeate of the UF_{VFA} from the thermophilic acidogenic fermenter, both concentrated in the RO_{RHA} plant, together with the fatty acid-rich RHL fraction, will feed an aerobic PHA-manufacturing reactor (RAPHA), in which a PHA-overproducing bacterium has been inoculated.

The synthesis and accumulation of PHA within PHA-overproducing bacteria occurs during the limitation of an essential nutrient and in the presence of an excess carbon source.

Working under these conditions, the PHA-overproducing bacterial strain will grow, storing PHA within same. Tracking the COD of the culture broth and the concentration of DO (dissolved oxygen) inside the aerobic reactor, together with the cell concentration in g/l, will be the main factors in controlling bacterial growth and accumulation of PHA therewithin. An increase in the concentration of DO coincides with a decrease in the value of the COD inside the RAPHA, therefore, the control of the metering of the three fractions which are fed to the RAPHA will be conditioned by the DO input, at which time the bacteria will generate PHA. TN (total nitrogen) and TP (total phosphorus) control will allow visualizing nitrogen and phosphorus sources, respectively, as limiting essential nutrient sources, particularly nitrogen in this case, taking into account the separation thereof in the form of Aa in the ion exchange columns. The limitation of an essential nutrient such as N or P, the excess input of a carbon source (COD) and the supply of oxygen allow the PHA-overproducing bacteria to accumulate PHA granules within them. PHA growth and purge will be monitored by tracking the TSS value.

Like in the case of the anaerobic VFA-generating reactor, phase E features, together with the aerobic PHA-manufacturing reactor (RAPHA), an ultrafiltration plant in a multitubular configuration, i.e., the UF_{RAPHA}, that allows separating the cell fraction inside the reactor.

The UF_{RAPHA} plant of the aerobic reactor will filter the contents of the aerobic reactor every time the contents of suspended solids, TSS, exceed the setpoint values. Exceeding the setpoint values indicates the presence of a sufficiently large bacterial population with sufficient PHA accumulation within same so as to carry out a PHA purge/extraction. The reading of COD, VFA and DO values allows the carbon source metered into the RAPHA to be regulated, while the reading of TSS allows the concentration of bacteria existing inside the reactor to be determined. When the TSS reading values inside the reactor exceed 25 g/l, the UF_{RAPHA} plant will be put in operation.

The UF_{RAPHA} plant generates two streams, a permeate stream which is rich in sugars/VFA and free of bacteria and a second solid fraction, at a maximum concentration of 25% v/v, which corresponds to the reject. The permeate will again be sent back to the aerobic PHA-manufacturing reactor, i.e., the RAPHA, whereas the reject, at a final concentration of 25% v/v, will feed a hydro-distiller identical to that of PHASE B in which, and working at 110°C and 5 bar, in batches, cell lysis, the release of PHA granules, and the redissolution of bacterial sugars and proteins will occur, along with the release of fatty acids that were part of the cell membranes of the bacteria. For the redissolution of the organic matter forming the cell wall of the bacteria, a mixture of sodium hydroxide/potassium hydroxide will be metered to the UF reject stream introduced into the hydro-distiller.

The hydro-distiller discharge, after the cell lysis process at 110°C and 5 bar, will feed a NUCHA filter in which all the solids, together with the PHA in the form of granules, will be retained in the lower part thereof, with the aqueous stream being filtered. The aqueous stream will combine with the load of the hydro-distiller of phase B which feeds the tricanter, generating RHA, RHS and RHL.

The solid retained in the NUCHA filter will be washed with an aqueous NaOH/KOH solution, recirculating it over the NUCHA filter itself, in order to separate the organic matter adhered to the PHA granules. The filtered washing stream will combine with the load of the hydro-distiller of phase B.

The washing process with recirculation will be extended until the PHA granules are perfectly cleaned of organic matter. At this point, a stream of hot, dry air originating from a generator will dry the PHA to moisture contents < 10% w/w. At this point, the NUCHA filter will be unloaded onto dry PHA collection bags.

## Claims

1. An organic matter treatment process for the production of polyhydroxyalkanoates (PHA) and compostable material, comprising the steps of:
a) provide residual organic matter,
b) homogenize-ground the residual organic matter mixture provided in the previous step,
c) introduce the ground residue into a hydro-distiller together with a mixture of organic and/or inorganic acids,
d) homogenize and then centrifuge the mixture obtained in the previous step to obtain an aqueous phase (RHA), a solid fraction (RHS) and an oily fraction (RHL),
e) filter the aqueous fraction (RHA) obtained in previous step d) by means of an ultrafiltration membrane system and sent to a system of ion exchange columns to obtain an eluate and a compostable fraction,
f) activate the solid fraction (RHS) obtained in the previous step d) with sludge from wastewater treatment plant (WWTP) and sent to feed an anaerobic biological reactor to produce volatile fatty acids (VFAs),
g) feed the oily fraction (RHL) obtained in step d), the eluate obtained from the ion exchange columns in step e) and a VFA-rich permeate of the ultrafiltration from the anaerobic biological reactor obtained in step f) to an aerobic PHA-reactor, wherein in step b) the residual organic matter used as raw material is homogenized - ground until a mush is formed, wherein the solids content has a size less than 100 microns and a variable composition depending on the mixture of solid and liquid component that has been made in the final recipe from the mixtures of fractions I to IV:
I.: Solid organic raw materials originating from supermarkets, hotels and restaurants, together with the organic matter from organic fraction in selective collection, waste from pruning and from the agricultural and agri-food industry,
II.: Liquid organic raw materials: whey, milk and dairy products, sugary and alcoholic beverages, cleaning water from industrial processes for the production of musts or juices,
III.: Sludge from WWTP and the output of anaerobic digesters, and
IV.: Livestock waste.

2. The organic matter treatment process for the production of PHA and compostable material according to claim 1, wherein the homogenized - ground residual organic matter is introduced into a hydro-distiller, together with a mixture of organic and/or inorganic acids, wherein the inorganic acids can be H₂SO₄, HCl, HNO₃, H₃PO₄, or mixture thereof, together with organic acids such as lactic acids, glycolic acid, sulfamic acid, or mixture thereof, including acid phosphonates or salts thereof, such as HEDP, ATMP, EDTMP, DTPMP, HDTMP, HEMPA or PBCT, acrylic acid homopolymers, acrylic and maleic acid polymers, or terpolymers working inside the hydro-distiller at a value of 1 ≤ pH ≤ 6, depending on the homogenized mixture of the initial recipe.

3. The organic matter treatment process for the production of PHA and compostable material according to claim 2, wherein the hydro-distiller is a reactor for steam/thermal oil which is made of stainless steel, heated with an outer jacket, or an inner jacket, or both, the inside of which is stirred by means of blades and having a reflux condenser for the steam generated therein, as well as an ultrasound system, which allows transforming:
a Fats and oils into fatty acids,
b Glycogen and starch into glucose, fructose, or lactose,
c Proteins into amino acids (Aa) and small peptide chains,
and wherein calcium and magnesium carbonates, sulfates and phosphates are solubilized through the addition of acidic solutions.

4. The organic matter treatment process for the production of PHA and compostable material according to claim 3, wherein the hydro-distiller works in batches with the following phases:
a Filling with the recipe according to claim 3
b Stirring the mixture
c Metering the acidic solution from a mixture of inorganic acids, organic acids, phosphonates and/or salts thereof, acrylic and/or acrylic/maleic acid derivatives and terpolymers, until reaching a value of 1 ≤ pH ≤ 6, which will depend on the recipe made.
d Heating the mixture at a temperature between 70ºC and 110ºC and subjecting same to cell lysis by means of providing heat and by means of the ultrasound system, respectively, until reaching pressure and temperature setpoint values, maintaining said pressure and temperature conditions for a time of 30 min to 120 min, sufficient to sterilize the mixture and release sugars, amino acids and fatty acids into the liquid medium.
e Pressurizing the mixture at the end of the heating time for 1 min to 15 min at a pressure that ranges from 2 bar to 5 bar.
f Finally, depressurizing the mixture.
g Emptying into HR: homogenization reactor
wherein the sterilized liquid discharge of the hydro-distiller is a broth rich in fatty acids, sugars, and amino acids together with dissolved mineral salts, being sulfates, carbonates and phosphates

5. The organic matter treatment process for the production of PHA and compostable material according to claim 4, wherein the discharge of the hydro-distiller is introduced into a homogenization reactor together with osmosis water in a broth:osmosis water ratio of 1:1 to 1:10, respectively.

6. The organic matter treatment process for the production of PHA and compostable material according to claim 5, wherein the mixture, hydro-distiller discharge-osmosis water, in a ratio of 1:1 to 1:10, is homogenized, pH-corrected, and finally treated by means of a tricanter centrifuge, obtaining three fractions:
a Oils: The least dense fraction - RHL fraction: Light fraction
b Aqueous phase: The intermediate phase - RHA fraction: Aqueous Fraction
c Solids: the densest phase - RHS fraction: Solid fraction

7. The organic matter treatment process for the production of PHA and compostable material according to claim 6, wherein the RHA fraction is filtered by means of an ultrafiltration membrane system, UF_{RHA}, with a molecular weight cut-off of 100,000 Daltons, obtaining two streams, a permeate stream, which has passed through the membranes and equivalent to 90-95% of the RHA fraction, which is sent to system of ion exchange columns, and a reject stream, which is the liquid fraction which has not passed through the membrane and is recirculated to the tricanter-feeding tank.

8. The organic matter treatment process for the production of PHA and compostable material according to claim 7, wherein the regeneration of the ion exchange columns is performed with KOH as base and with H₂SO₄ as acid, where there is obtained, from the regeneration of the ion exchange columns, a root biostimulant made up of Aa + inorganic salts rich in potassium and phosphorus, which are crystallized by means of using an aerosol emission crystallizer.

9. The organic matter treatment process for the production of PHA and compostable material according to claim 7, wherein the eluate of the ion exchange columns corresponds with an aqueous liquid fraction which is rich in sugars and has a low conductivity, which will be used to feed the anaerobic biological PHA-manufacturing reactor, i.e., the RAPHA.

10. The organic matter treatment process for the production of PHA and compostable material according to claim 6, wherein the RHS fraction is used to feed an anaerobic reactor for generating volatile fatty acids (VFA) by means of an acidogenic thermophilic fermentation process, wherein the bacterial inoculum originates from the active sludge from WWTP (wastewater treatment plant) and wherein the control of the growth of VFA-producing bacterial strains is performed by means of monitoring COD (chemical oxygen demand) or TOC (total organic carbon), pH, T° (temperature), TSS (total solids in suspension), TN (total nitrogen), TP (total phosphorus), and VFA (volatile fatty acid) parameters, and bacterial fraction is purged from the inside of the reactor by means of an ultra-filtration (UF_{VFA})plant in a multitubular configuration.

11. The organic matter treatment process for the production of PHA and compostable material according to claim 10, further comprising the steps of:
a) wherein the contents of the anaerobic biological VFA-generating reactor (acidogenic thermophilic fermenter) is continuously filtered by means of a ultrafiltration (UF_{VFA}) plant in a multitubular configuration, from which two streams are obtained, a permeate stream, free of bacteria and loaded with VFA + water + mineral salts and another reject stream, in which the bacterial load is concentrated up to a maximum solid contents of 50% v/v,
b) the permeate fraction (RHA) from the UF_{VFA} plant is corrected to a value of 7 ≤ pH ≤ 12 and then subjected to a concentration process using a reverse osmosis (RO_{RHA}) membrane system, which generates two streams, a water stream, i.e., the permeate, that returns to the anaerobic VFA-generating reactor (thermophilic acidogenic fermenter) and another reject stream, in which VFAs are found in the form of sodium or potassium salts + inorganic salts, which will be used to feed the PHA-manufacturing plant,
c) the reject of the UF_{VFA} is recirculated over the anaerobic VFA-generating reactor, as long as the concentration of solids inside the reactor does not exceed 25 g/l, wherein the reject of the UF_{VFA} plant is sent directly for the thermal drying of sludge in order to manufacture biostimulant: organic fraction,
d) this purging process of the previous step is maintained until the value of solids inside the anaerobic fermentation reactor drops below 12 g/l, at which time all the reject of the UF_{VFA} plant is redirected again to the anaerobic VFA-generating reactor.

12. The organic matter treatment process for the production of PHA and compostable material according to claim 9, wherein there is inoculated in the anaerobic PHA-manufacturing reactor, i.e., the RAPHA, a PHA-overproducing bacterium which is fed with three fractions:
a The eluate from the ion exchange columns corresponding with an aqueous liquid fraction which is rich in sugars and has a low conductivity,
b The RHL fraction obtained in the tricanter, as the lightest fraction in the centrifugation separation and corresponding to the fatty acid fraction, and
c The reject of the RO plant which, fed from the permeate of the multitubular UF_{VFA} of the anaerobic reactor, generates a stream of VFA in the form of sodium salts + inorganic salts, in aqueous solution,
and wherein the control of the growth and accumulation of PHA in the bacterial fraction, together with the control of the feeding of each of the three previous fractions into the RAPHA, is carried out by continuously measuring the COD (chemical oxygen demand) or TOC (total organic carbon), pH, T° (temperature), DO (dissolved oxygen), TSS (total solids in suspension), TN (total nitrogen), TP (total phosphorus) and VFA parameters, whereas the PHA-loaded bacterial fraction is purged from the inside of the reactor by means of an UF_{RAPHA} plant in a multitubular configuration.

13. The organic matter treatment process for the production of PHA and compostable material according to claim 12, further comprising the steps of:
a) once the suspended solids setpoint value of 25 g/l is reached the contents of the aerobic PHA-generating biological reactor, i.e., the RAPHA, is continuously filtered by means of a ultrafiltration (UF_{RAPHA}) plant in a multitubular configuration to obtain two streams, a permeate stream, free of bacteria and loaded with VFA, sugars, fatty acids and mineral salts in aqueous suspension and another reject stream, in which the bacterial load is concentrated up to a maximum solid contents of 25 % v/v,
b) the permeate fraction is sent continuously back to the aerated PHA-manufacturing biological reactor, i.e., the RAPHA, whereas the reject fraction will be purged from the system, in its entirety up to hydro-distiller, for the extraction of PHA from PHA-overproducing bacteria until the concentration of solids in the RAPHA drops below 12 g/l, at which time the UF_{RAPHA} plant will stop operating.

14. The organic matter treatment process for the production of PHA and compostable material according to claim 12, wherein the hydro-distiller, which is fed with what is purged from UF_{RAPHA}, works in batches with the following phases:
a Filling with the reject of UF_{RAPHA}
b Stirring the mixture
c Metering the basic solution from a mixture of NaOH/KOH, until reaching a value of 11 ≤ pH ≤ 14.
d Heating the mixture and subjecting same to cell lysis by means of providing heat and by means of the ultrasound system, respectively, until reaching pressure and temperature setpoint values, maintaining said pressure and temperature conditions for a time of 30 min to 120 min, sufficient to sterilize the mixture and release the PHA contained in the PHA-overproducing bacteria.
e Pressurizing the mixture at the end of the heating time for 1 min to 15 min.
f Finally, depressurizing the mixture.
g Emptying over a pressure and vacuum filter.
wherein the PHA-rich sterilized liquid discharge of the hydro-distiller can be separated from the liquid fraction as a result of the filter.

15. The organic matter treatment process for the production of PHA and compostable material according to claim 13, wherein the PHA retained in the filter is washed with a KOH/NaOH stream until it is free of organic matter, followed by a wash with osmosis water and finally drying with a stream of hot and dry air.

16. The organic matter treatment process for the production of PHA and compostable material according to claim 8, wherein the root biostimulant is made up of:
a an inorganic fraction rich in potassium and phosphorus and another organic fraction rich in nitrogen originating from amino acids, originating from the regeneration of the ion exchange columns.
b an organic matter fraction originating from what is purged from the reject of the UF_{VFA} of the acidogenic thermophilic VFA-generating fermenter,
and wherein the mixtures of the two fractions are dehydrated and dried by means of a solid dryer with hot air.

## Patentansprüche

1. Verfahren zur Behandlung von organischen Stoffen zur Herstellung von Polyhydroxyalkanoaten (PHA) und kompostierbarem Material, umfassend die folgenden Schritte:
a) Bereitstellen von organischen Reststoffen,
b) Homogenisieren-Zerkleinern des im vorherigen Schritt bereitgestellten Gemischs aus organischen Reststoffen,
c) Einführen des zerkleinerten Rückstandes in einen Hydro-Destillator zusammen mit einem Gemisch aus organischen und/oder anorganischen Säuren,
d) Homogenisieren und dann Zentrifugieren des im vorherigen Schritt erhaltenen Gemisches, um eine wässrige Phase (RHA), eine Feststoff-Fraktion (RHS) und eine ölige Fraktion (RHL) zu erhalten,
e) Filtern der im vorherigen Schritt d) erhaltenen wässrigen Fraktion (RHA) mittels eines Ultrafiltrationsmembransystems und Weiterleiten an ein System von Ionenaustauschersäulen, um ein Eluat und eine kompostierbare Fraktion zu erhalten,
f) Aktivieren der im vorherigen Schritt d) erhaltenen Feststoff-Fraktion (RHS) mit Schlamm aus einem Klärwerk (WWTP) und Weiterleiten zur Einspeisung in einen anaeroben biologischen Reaktor, um flüchtige Fettsäuren (VFAs) zu erzeugen,
g) Einspeisen der in Schritt d) erhaltenen öligen Fraktion (RHL), des in Schritt e) aus den Ionenaustauschersäulen erhaltenen Eluats und eines in Schritt f) erhaltenen VFA-reichen Permeats der Ultrafiltration aus dem anaeroben biologischen Reaktor in einen aeroben PHA-Reaktor,
wobei in Schritt b) der als Rohstoff verwendete organische Reststoff homogenisiert - zerkleinert wird, bis ein Brei entsteht, wobei der Feststoffgehalt eine Größe von weniger als 100 Mikrometer und eine variable Zusammensetzung in Abhängigkeit von dem Gemisch aus fester und flüssiger Komponente aufweist, das in der endgültigen Rezeptur aus den Gemischen der Fraktionen I bis IV hergestellt wurde:
I.: Feste organische Rohstoffe, die aus Supermärkten, Hotels und Restaurants stammen, zusammen mit den organischen Stoffen aus der organischen Fraktion in der selektiven Sammlung, Abfällen aus dem Baumschnitt und aus der Landwirtschafts- und Agrarlebensmittelindustrie,
II.: Flüssige organische Rohstoffe: Molke, Milch und Molkereiprodukte, zuckerhaltige und alkoholische Getränke, Reinigungswasser aus industriellen Verfahren zur Herstellung von Mosten oder Säften,
III.: Schlamm aus dem WWTP und der Ausgabe von anaeroben Digerierapparaten, und
IV.: Viehzuchtabfälle.

2. Verfahren zur Behandlung von organischen Stoffen zur Herstellung von PHA und kompostierbarem Material nach Anspruch 1, wobei der homogenisierte - zerkleinerte - organische Reststoff in einen Hydro-Destillator eingeführt wird, zusammen mit einem Gemisch aus organischen und/oder anorganischen Säuren, wobei die anorganischen Säuren H₂SO₄, HCl, HNO₃, H₃PO₄ oder ein Gemisch davon sein können, zusammen mit organischen Säuren wie Milchsäuren, Glykolsäure, Sulfaminsäure, oder einem Gemisch davon, einschließlich saurer Phosphonate oder Salzen davon, wie HEDP, ATMP, EDTMP, DTPMP, HDTMP, HEMPA oder PBCT, Acrylsäurehomopolymeren, Acryl- und Maleinsäurepolymeren oder Terpolymeren, die innerhalb des Hydro-Destillators bei einem Wert von 1 ≤ pH ≤ 6 arbeiten, in Abhängigkeit von dem homogenisierten Gemisch der Ausgangsrezeptur.

3. Verfahren zur Behandlung von organischen Stoffen zur Herstellung von PHA und kompostierbarem Material nach Anspruch 2, wobei der Hydro-Destillator ein Reaktor für Dampf/Thermalöl ist, der aus rostfreiem Stahl hergestellt ist, mit einem Außenmantel oder einem Innenmantel oder beidem erwärmt wird, dessen Inneres mittels Flügeln gerührt wird und einen Rückflusskondensator für den darin generierten Dampf sowie ein Ultraschallsystem aufweist, das das Umwandeln von Folgendem ermöglicht:
a Fette und Öle in Fettsäuren,
b Glykogen und Stärke in Glucose, Fructose oder Lactose,
c Proteine in Aminosäuren (Aa) und kleine Peptidketten,
und wobei Calcium- und Magnesiumcarbonate, -sulfate und -phosphate durch die Addition von sauren Lösungen aufgelöst werden.

4. Verfahren zur Behandlung von organischen Stoffen zur Herstellung von PHA und kompostierbarem Material nach Anspruch 3, wobei der Hydro-Destillator in Chargen mit den folgenden Phasen arbeitet:
a Füllen mit der Rezeptur nach Anspruch 3,
b Rühren des Gemisches,
c Dosieren der sauren Lösung aus einem Gemisch aus anorganischen Säuren, organischen Säuren, Phosphonaten und/oder Salzen davon, Acryl- und/oder Acryl-/Maleinsäurederivaten und Terpolymeren, bis ein Wert von 1 ≤ pH ≤ 6 erreicht ist, der von der hergestellten Rezeptur abhängt,
d Erwärmen des Gemisches auf eine Temperatur zwischen 70 °C und 110 °C und Unterziehen desselben einer Zelllyse jeweils mittels Bereitstellen von Wärme und mittels des Ultraschallsystems bis zum Erreichen von Druck- und Temperatursollwerten, wobei die Druck- und Temperaturbedingungen für einen Zeitraum von 30 min bis 120 min aufrechterhalten werden, ausreichend, um das Gemisch zu sterilisieren und Zucker, Aminosäuren und Fettsäuren in das flüssige Medium freizusetzen,
e Druckbeaufschlagen des Gemischs am Ende des Zeitraums der Erwärmung für 1 min bis 15 min bei einem Druck im Bereich von 2 bar bis 5 bar,
f Schließlich Druckentlasten des Gemisches,
g Entleeren in den HR: Homogenisierungsreaktor.
wobei der sterilisierte flüssige Ausfluss des Hydro-Destillators eine Brühe ist, die reich an Fettsäuren, Zuckern und Aminosäuren zusammen mit gelösten Mineralsalzen ist, wobei es sich um Sulfate, Carbonate und Phosphate handelt.

5. Verfahren zur Behandlung von organischen Stoffen zur Herstellung von PHA und kompostierbarem Material nach Anspruch 4, wobei der Ausfluss des Hydro-Destillators zusammen mit Osmosewasser in einem Verhältnis von Brühe:Osmosewasser von jeweils 1:1 bis 1:10 in einen Homogenisierungsreaktor eingeführt wird.

6. Verfahren zur Behandlung von organischen Stoffen zur Herstellung von PHA und kompostierbarem Material nach Anspruch 5, wobei das Gemisch, Hydro-Distiller-Ausfluss-Osmosewasser, in einem Verhältnis von 1:1 bis 1:10 homogenisiert, pH-korrigiert und schließlich mittels einer Tricanter-Zentrifuge behandelt wird, wobei drei Fraktionen erhalten werden:
a Öle: Die Fraktion mit der geringsten Dichte - RHL-Fraktion: Leichte Fraktion,
b Wässrige Phase: Die mittlere Phase - RHA-Fraktion: Wässrige Fraktion,
c Feststoffe: Die dichteste Phase - RHS-Fraktion: Feststoff-Fraktion.

7. Verfahren zur Behandlung von organischen Stoffen zur Herstellung von PHA und kompostierbarem Material nach Anspruch 6, wobei die RHA-Fraktion mittels eines Ultrafiltrationsmembransystems, UF_{RHA}, mit einem Molekulargewichts-Cut-off von 100.000 Dalton gefiltert wird, wobei zwei Ströme erhalten werden, ein Permeatstrom, der die Membranen passiert hat und 90-95 % der RHA-Fraktion entspricht, der zu einem System von Ionenaustauschersäulen weitergeleitet wird, und ein Ausschussstrom, der die flüssige Fraktion ist, die die Membran nicht passiert hat und in den Tricanter-Einspeisetank zurückgeleitet wird.

8. Verfahren zur Behandlung von organischen Stoffen zur Herstellung von PHA und kompostierbarem Material nach Anspruch 7, wobei die Regeneration der Ionenaustauschersäulen mit KOH als Base und mit H₂SO₄ als Säure durchgeführt wird, wobei aus der Regeneration der Ionenaustauschersäulen ein Wurzelbiostimulans aus Aa + anorganischen Salzen, die reich an Kalium und Phosphor sind, hergestellt wird, die mittels der Verwendung eines Aerosol-Emissionskristallisators kristallisiert werden.

9. Verfahren zur Behandlung von organischen Stoffen zur Herstellung von PHA und kompostierbarem Material nach Anspruch 7, wobei das Eluat der Ionenaustauschersäulen einer wässrigen flüssigen Fraktion entspricht, die reich an Zuckern ist und eine niedrige Leitfähigkeit aufweist, die zur Speisung des anaeroben biologischen PHA-Herstellungsreaktors, d. h. des RAPHA, verwendet wird.

10. Verfahren zur Behandlung von organischen Stoffen zur Herstellung von PHA und kompostierbarem Material nach Anspruch 6, wobei die RHS-Fraktion zur Speisung eines anaeroben Reaktors verwendet wird, um flüchtige Fettsäuren (VFA) mittels eines acidogenen thermophilen Fermentationsverfahrens zu generieren, wobei das bakterielle Inokulum aus dem aktiven Schlamm des WWTP (Klärwerks) stammt und wobei die Kontrolle des Wachstums der VFAproduzierenden Bakterienstämme mittels des Überwachens der Parameter COD (chemischer Sauerstoffbedarf) oder TOC (gesamter organischer Kohlenstoff), pH, T° (Temperatur), TSS (Gesamtfeststoffe in Suspension), TN (Gesamtstickstoff), TP (Gesamtphosphor) und VFA (flüchtige Fettsäuren) durchgeführt wird und die bakterielle Fraktion aus dem Inneren des Reaktors mittels einer Ultrafiltrations(UF_{VFA})-Anlage in einer multitubulären Konfiguration abgelassen wird.

11. Verfahren zur Behandlung von organischen Stoffen zur Herstellung von PHA und kompostierbarem Material nach Anspruch 10, das ferner die folgenden Schritte umfasst:
a) der Inhalt des anaeroben biologischen VFA-generierenden Reaktors (acidogener thermophiler Fermenter) wird kontinuierlich mittels einer Ultrafiltrations(UF_{VFA})-Anlage in einer multitubulären Konfiguration gefiltert, aus der zwei Ströme erhalten werden, ein bakterienfreier Permeatstrom, der mit VFA + Wasser + Mineralsalzen beladen ist, und ein weiterer Ausschussstrom, in dem die bakterielle Belastung bis zu einem maximalen Feststoffgehalt von 50 % v/v konzentriert ist,
b) die Permeatfraktion (RHA) aus der UF_{VFA}-Anlage wird auf einen Wert von 7 ≤ pH ≤ 12 korrigiert und dann einem Konzentrationsverfahren unter Verwendung eines Umkehrosmose(RO_{RHA})-Membransystems unterzogen, bei dem zwei Ströme generiert werden, ein Wasserstrom, d. h., das Permeat, das in den anaeroben VFA-generierenden Reaktor (thermophiler acidogener Fermenter) zurückfließt, und einen weiteren Ausschussstrom, in dem sich VFAs in Form von Natrium- oder Kaliumsalzen + anorganischen Salzen befinden, die zur Speisung der PHA-Herstellungsanlage verwendet werden,
c) der Ausschuss der UF_{VFA} wird über den anaeroben VFA-generierenden Reaktor zurückgeleitet, solange die Feststoffkonzentration innerhalb des Reaktors 25 g/l nicht übersteigt, wobei der Ausschuss der UF_{VFA}-Anlage direkt zur thermischen Trocknung des Schlamms weitergeleitet wird, um ein Biostimulans: organische Fraktion herzustellen,
d) dieser Ablassprozess des vorherigen Schritts wird so lange aufrechterhalten, bis der Wert der Feststoffe innerhalb des anaeroben Fermentationsreaktors unter 12 g/l fällt, woraufhin der gesamte Ausschuss der UF_{VFA}-Anlage erneut in den anaeroben VFA-generierenden Reaktor geleitet wird.

12. Verfahren zur Behandlung von organischen Stoffen zur Herstellung von PHA und kompostierbarem Material nach Anspruch 9, wobei in den anaeroben PHA-Herstellungsreaktor, d. h. den RAPHA, ein PHA-überproduzierendes Bakterium inokuliert wird, das mit drei Fraktionen gespeist wird:
a das Eluat aus den Ionenaustauschersäulen, das einer wässrigen flüssigen Fraktion entspricht, die reich an Zuckern ist und eine niedrige Leitfähigkeit aufweist,
b die RHL-Fraktion, die im Tricanter als leichteste Fraktion bei der Zentrifugationstrennung erhalten wird und der Fettsäurefraktion entspricht, und
c der Ausschuss der RO-Anlage, der, gespeist aus dem Permeat der multitubulären UF_{VFA} des anaeroben Reaktors, einen Strom von VFA in Form von Natriumsalzen + anorganischen Salzen, in wässriger Lösung generiert,
und wobei die Kontrolle des Wachstums und der Anreicherung von PHA in der bakteriellen Fraktion zusammen mit der Kontrolle der Einspeisung jeder der drei vorherigen Fraktionen in den RAPHA durch das kontinuierliche Messen der Parameter COD (chemischer Sauerstoffbedarf) oder TOC (gesamter organischer Kohlenstoff), pH, T° (Temperatur), DO (gelöster Sauerstoff), TSS (Gesamtfeststoffe in Suspension), TN (Gesamtstickstoff), TP (Gesamtphosphor) und VFA durchgeführt wird, während die mit PHA beladene bakterielle Fraktion aus dem Inneren des Reaktors mittels einer UF_{RAPHA}-Anlage in einer multitubulären Konfiguration abgelassen wird.

13. Verfahren zur Behandlung von organischen Stoffen zur Herstellung von PHA und kompostierbarem Material nach Anspruch 12, das ferner die folgenden Schritte umfasst:
a) sobald der Sollwert für suspendierte Feststoffe von 25 g/l erreicht ist, wird der Inhalt des aeroben PHAgenerierenden biologischen Reaktors, d. h. des RAPHA, kontinuierlich mittels einer Ultrafiltrations(UF_{RAPHA})-Anlage in einer multitubulären Konfiguration gefiltert, um zwei Ströme zu erhalten, einen bakterienfreien Permeatstrom, der mit VFA, Zuckern, Fettsäuren und Mineralsalzen in wässriger Suspension beladen ist, und einen weiteren Ausschussstrom, in dem die bakterielle Belastung bis zu einem maximalen Feststoffgehalt von 25 % v/v konzentriert ist,
b) die Permeatfraktion wird kontinuierlich in den belüfteten biologischen PHA-Herstellungsreaktor, d. h. den RAPHA, zurückgeleitet, während die Ausschussfraktion zur Extraktion von PHA aus den PHA-überproduzierenden Bakterien bis zum Hydro-Destillator vollständig aus dem System abgelassen wird, bis die Feststoffkonzentration im RAPHA unter 12 g/l fällt, woraufhin die UF_{RAPHA}-Anlage ihren Betrieb beendet.

14. Verfahren zur Behandlung von organischen Stoffen zur Herstellung von PHA und kompostierbarem Material nach Anspruch 12, wobei der Hydro-Destillator, der mit dem aus UF_{RAPHA} abgelassenem Material gespeist wird, in Chargen mit den folgenden Phasen arbeitet:
a Füllen mit dem Ausschuss von UF_{RAPHA},
b Rühren des Gemisches,
c Dosieren der basischen Lösung aus einem Gemisch aus NaOH/KOH, bis ein Wert von 11 ≤ pH ≤ 14 erreicht ist,
d Erwärmen des Gemisches und Unterziehen desselben einer Zelllyse jeweils mittels Bereitstellen von Wärme und mittels des Ultraschallsystems bis zum Erreichen von Druck- und Temperatursollwerten, wobei die Druck- und Temperaturbedingungen für einen Zeitraum von 30 min bis 120 min aufrechterhalten werden, ausreichend, um das Gemisch zu sterilisieren und das in den PHA-überproduzierenden Bakterien enthaltene PHA freizusetzen,
e Druckbeaufschlagen des Gemischs am Ende des Zeitraums der Erwärmung für 1 min bis 15 min,
f Schließlich Druckentlasten des Gemisches,
g Entleeren über einen Druck- und Vakuumfilter.
wobei der PHA-reiche sterilisierte flüssige Ausfluss des Hydro-Destillators als Ergebnis des Filters von der flüssigen Fraktion getrennt werden kann.

15. Verfahren zur Behandlung von organischen Stoffen zur Herstellung von PHA und kompostierbarem Material nach Anspruch 13, wobei das im Filter zurückgehaltene PHA mit einem KOH/NaOH-Strom gewaschen wird, bis es frei von organischen Stoffen ist, gefolgt von einer Wäsche mit Osmosewasser und schließlich einer Trocknung mit einem Strom aus heißer und trockener Luft.

16. Verfahren zur Behandlung von organischen Stoffen zur Herstellung von PHA und kompostierbarem Material nach Anspruch 8, wobei das Wurzelbiostimulans aus Folgendem besteht:
a einer anorganischen Fraktion, die reich an Kalium und Phosphor ist, und einer anderen organischen Fraktion, die reich an aus Aminosäuren stammendem Stickstoff ist und aus der Regeneration der Ionenaustauschersäulen stammt,
b eine organische Fraktion, die aus dem stammt, was aus dem Ausschuss der UF_{VFA} des acidogenen thermophilen VFA-generierenden Fermenters abgelassen wird,
wobei die Gemische der beiden Fraktionen mittels eines Feststofftrockners mit heißer Luft dehydriert und getrocknet werden.

## Revendications

1. Procédé de traitement de matière organique pour la production de polyhydroxyalcanoates (PHA) et de matériaux compostables, comprenant les étapes consistant à :
a) fournir de la matière organique résiduelle,
b) homogénéiser et broyer le mélange de matière organique résiduelle obtenu à l'étape précédente,
c) introduire le résidu broyé dans un hydrodistillateur conjointement avec un mélange d'acides organiques et/ou inorganiques,
d) homogénéiser puis centrifuger le mélange obtenu à l'étape précédente pour obtenir une phase aqueuse (RHA), une fraction solide (RHS) et une fraction huileuse (RHL),
e) filtrer la fraction aqueuse (RHA) obtenue à l'étape précédente d) au moyen d'un système de membrane d'ultrafiltration et l'envoyer dans un système de colonnes d'échange d'ions pour obtenir un éluat et une fraction compostable,
f) activer la fraction solide (RHS) obtenue à l'étape précédente d) avec des boues de station d'épuration des eaux usées (STEP) et l'envoyer pour alimenter un réacteur biologique anaérobie pour produire des acides gras volatils (AGV),
g) introduire la fraction huileuse (RHL) obtenue à l'étape d), l'éluat obtenu à partir des colonnes d'échange d'ions à l'étape e) et un perméat riche en AGV de l'ultrafiltration du réacteur biologique anaérobie obtenu à l'étape f) vers un réacteur à PHA aérobie,
dans lequel à l'étape b) la matière organique résiduelle utilisée comme matière première est homogénéisée - broyée jusqu'à ce qu'une bouillie soit formée, dans lequel la teneur en solides a une taille inférieure à 100 microns et une composition variable en fonction du mélange de composants solides et liquides qui a été réalisé dans la recette finale à partir des mélanges de fractions I à IV :
I. : Matières premières organiques solides provenant de supermarchés, d'hôtels et de restaurants, conjointement avec la matière organique de fraction organique en collecte sélective, de déchets d'élagage et de l'industrie agricole et agroalimentaire,
II. : Matières premières organiques liquides : petit lait, lait et produits laitiers, boissons sucrées et alcoolisées, eaux de nettoyage de processus industriels pour la production de moûts ou de jus,
III. : Boues de STEP et la sortie des digesteurs anaérobies, et
IV : Déchets d'élevage.

2. Procédé de traitement de matière organique pour la production de PHA et de matière compostable selon la revendication 1, dans lequel la matière organique résiduelle homogénéisée et broyée est introduite dans un hydrodistillateur, conjointement avec un mélange d'acides organiques et/ou inorganiques, dans lequel les acides inorganiques peuvent être H₂SO₄, HCl, HNO₃, H₃PO₄, ou leur mélange, conjointement avec des acides organiques tels que les acides lactiques, l'acide glycolique, l'acide sulfamique ou leur mélange, y compris les phosphonates acides ou leurs sels, tels que l'HEDP, l'ATMP, l'EDTMP, le DTPMP, l'HDTMP, l'HEMPA ou le PBCT, les homopolymères de l'acide acrylique, les polymères d'acide acrylique et maléique, ou les terpolymères travaillant à l'intérieur de l'hydrodistillateur à une valeur de 1 ≤ pH ≤ 6, en fonction du mélange homogénéisé de la recette initiale.

3. Procédé de traitement de matière organique pour la production de PHA et de matière compostable selon la revendication 2, dans lequel l'hydrodistillateur est un réacteur pour vapeur/huile thermique qui est fait en acier inoxydable, chauffé avec une enveloppe extérieure, ou une enveloppe intérieure, ou les deux, dont l'intérieur est agité au moyen de pales et ayant un condenseur à reflux pour la vapeur générée à l'intérieur de celui-ci, ainsi qu'un système à ultrasons, qui permet de transformer :
a Les graisses et les huiles en acides gras,
b Le glycogène et l'amidon en glucose, fructose, ou lactose,
c Les protéines en acides aminés (Aa) et les petites chaînes peptidiques,
et dans lequel les carbonates de calcium et de magnésium, les sulfates et les phosphates sont solubilisés à travers l'ajout de solutions acides.

4. Procédé de traitement de matière organique pour la production de PHA et de matière compostable selon la revendication 3, dans lequel l'hydrodistillateur travaille par lots avec les phases suivantes :
a Remplir avec la recette selon la revendication 3,
b Remuer le mélange,
c Doser la solution acide à partir d'un mélange d'acides inorganiques, d'acides organiques, de phosphonates et/ou de leurs sels, de dérivés d'acide acrylique et/ou acrylique/maléique et de terpolymères, jusqu'à atteindre une valeur de 1 ≤ pH ≤ 6, qui dépend de la recette réalisée,
d Chauffer le mélange à une température entre 70 °C et 110 °C et soumettre celui-ci à une lyse cellulaire au moyen de la fourniture de chaleur et au moyen du système à ultrasons, respectivement, jusqu'à ce que les valeurs de consigne de la pression et de la température soient atteintes, maintenir lesdites conditions de pression et de température pendant une durée de 30 à 120 minutes, suffisante pour stériliser le mélange et libérer les sucres, les acides aminés et les acides gras dans le milieu liquide,
e Pressuriser le mélange à la fin de la durée de chauffage pendant 1 min à 15 min à une pression qui va de 2 bar à 5 bar,
f Enfin, dépressuriser le mélange,
g Vider dans le RH : réacteur d'homogénéisation.
dans lequel la décharge de liquide stérilisé de l'hydrodistillateur est un bouillon riche en acides gras, en sucres et en acides aminés ainsi qu'en sels minéraux dissous, étant des sulfates, des carbonates et des phosphates.

5. Procédé de traitement de matière organique pour la production de PHA et de matière compostable selon la revendication 4, dans lequel la décharge de l'hydrodistillateur est introduite dans un réacteur d'homogénéisation conjointement avec l'eau d'osmose dans un rapport bouillon/eau d'osmose de 1:1 à 1:10, respectivement.

6. Procédé de traitement de matière organique pour la production de PHA et de matière compostable selon la revendication 5, dans lequel le mélange, décharge d'hydro-distillateur - eau d'osmose, dans un rapport de 1:1 à 1:10, est homogénéisé, corrigé en pH, et enfin traité au moyen d'une centrifugeuse tricanter, obtenant trois fractions :
a Huiles : La fraction la moins dense - fraction RHL : Fraction légère,
b Phase aqueuse : La phase intermédiaire - fraction RHA : Fraction aqueuse,
c Solides : la phase la plus dense - fraction RHS : Fraction solide.

7. Procédé de traitement de matière organique pour la production de PHA et de matière compostable selon la revendication 6, dans lequel la fraction RHA est filtrée au moyen d'un système de membrane d'ultrafiltration, UF_{RHA}, avec un seuil de coupure moléculaire de 100 000 daltons, obtenant deux flux, un flux de perméat, qui a traversé les membranes et qui équivaut à 90-95 % de la fraction RHA, qui est envoyé dans un système de colonnes d'échange d'ions, et un flux de rejet, qui est la fraction liquide qui n'a pas traversé la membrane et qui est recirculée vers le réservoir d'alimentation de tricanter.

8. Procédé de traitement de matière organique pour la production de PHA et de matière compostable selon la revendication 7, dans lequel la régénération des colonnes d'échange d'ions est effectuée avec KOH comme base et avec H₂SO₄ comme acide, où l'on obtient, à partir de la régénération des colonnes d'échange d'ions, un biostimulant racinaire composé d'Aa + de sels inorganiques riches en potassium et en phosphore, qui sont cristallisés au moyen de l'utilisation d'un cristalliseur à émission d'aérosol.

9. Procédé de traitement de matière organique pour la production de PHA et de matière compostable selon la revendication 7, dans lequel l'éluat des colonnes d'échange d'ions correspond à une fraction liquide aqueuse qui est riche en sucres et a une faible conductivité, qui servira à alimenter le réacteur biologique anaérobie de fabrication de PHA, à savoir, le RAPHA.

10. Procédé de traitement de matière organique pour la production de PHA et de matière compostable selon la revendication 6, dans lequel la fraction RHS est utilisée pour alimenter un réacteur anaérobie permettant de générer des acides gras volatils (AGV) au moyen d'un processus de fermentation thermophile acidogène, dans lequel l'inoculum bactérien est issu des boues actives de STEP (station d'épuration des eaux usées) et dans lequel le contrôle de la croissance de souches bactériennes productrices d'AGV est effectué au moyen de la surveillance de la DCO (demande chimique en oxygène) ou du COT (carbone organique total), du pH, de la T° (température), du TSS (total des solides en suspension), du TN (azote total), des paramètres de PT (phosphore total) et d'AGV (acides gras volatils), et la fraction bactérienne est purgée de l'intérieur du réacteur au moyen d'une installation d'ultrafiltration (UF_{AGV}) dans une configuration multitubulaire.

11. Procédé de traitement de matière organique pour la production de PHA et de matière compostable selon la revendication 10, comprenant en outre les étapes de :
a) dans lequel le contenu du réacteur biologique anaérobie générateur d'AGV (fermenteur thermophile acidogène) est filtré en continu au moyen d'une installation d'ultrafiltration (UF_{AGV}) dans une configuration multitubulaire, à partir de laquelle deux flux sont obtenus, un flux de perméat, exempt de bactéries et chargé d'AGV, d'eau, de sels minéraux et d'un autre flux de rejet, où la charge bactérienne est concentrée jusqu'à une teneur maximale en solides de 50 % v/v,
b) la fraction de perméat (RHA) provenant de l'usine UF_{AGV} est corrigée à une valeur de 7 ≤ pH ≤ 12, puis soumise à un processus de concentration à l'aide d'un système de membrane d'osmose inverse (RO_{RHA}), qui génère deux flux, un cours d'eau, à savoir, le perméat, qui retourne au réacteur anaérobie générateur d'AGV (fermenteur acidogène thermophile) et un autre flux de rejet, où les AGV se trouvent sous la forme de sels de sodium ou de potassium + sels inorganiques, qui serviront à alimenter l'usine de fabrication de PHA,
c) le rejet de l'UF_{AGV} est recirculé dans le réacteur anaérobie générateur d'AGV, tant que la concentration de solides à l'intérieur du réacteur ne dépasse pas 25 g/l, dans lequel le rejet de l'installation UF_{AGV} est envoyé directement pour le séchage thermique de boues afin de fabriquer un biostimulant : fraction organique,
d) ce processus de purge de l'étape précédente est maintenu jusqu'à ce que la valeur de solides à l'intérieur du réacteur de fermentation anaérobie tombe en dessous de 12 g/l, à ce moment-là tous les rejets de l'installation d'UF_{AGV} sont redirigés vers le réacteur anaérobie générateur d'AGV.

12. Procédé de traitement de matière organique pour la production de PHA et de matière compostable selon la revendication 9, dans lequel on inocule dans le réacteur anaérobie de fabrication de PHA, à savoir, le RAPHA, une bactérie surproductrice de PHA qui est alimentée avec trois fractions :
a L'éluat provenant des colonnes d'échange d'ions correspondant à une fraction liquide aqueuse qui est riche en sucres et a une faible conductivité,
b La fraction RHL obtenue dans le tricanter, comme la fraction la plus légère dans la séparation par centrifugation et correspondant à la fraction d'acides gras, et
c Le rejet de l'installation d'osmose inverse qui, alimentée par le perméat de l'UF_{AGV} multitubulaire du réacteur anaérobie, génère un flux d'AGV sous forme de sels de sodium + sels inorganiques, en solution aqueuse,
et dans lequel le contrôle de la croissance et de l'accumulation de PHA dans la fraction bactérienne, ainsi que le contrôle de l'alimentation de chacune des trois fractions précédentes dans le RAPHA, est mise en oeuvre en mesurant en continu la DCO (demande chimique en oxygène) ou le COT (carbone organique total), le pH, la T° (température), le DO (oxygène dissous), le TSS (total des solides en suspension), le TN (azote total), les paramètres TP (phosphore total) et AGV, tandis que la fraction bactérienne chargée en PHA est purgée de l'intérieur du réacteur au moyen d'une installation UF_{RAPHA} dans une configuration multitubulaire.

13. Procédé de traitement de matière organique pour la production de PHA et de matière compostable selon la revendication 12, comprenant en outre les étapes de :
a) lorsque la valeur de consigne de solides en suspension de 25 g/l est atteinte, le contenu du réacteur biologique aérobie générateur de PHA, à savoir, le RAPHA, est filtrée en continu au moyen d'une installation d'ultrafiltration (UF_{RAPHA}) dans une configuration multitubulaire pour obtenir deux flux, un flux de perméat, exempt de bactéries et chargé d'AGV, de sucres, d'acides gras et de sels minéraux en suspension aqueuse et un autre flux de rejet, où la charge bactérienne est concentrée jusqu'à une teneur maximale en solides de 25 % v/v,
b) la fraction de perméat est renvoyée en continu dans le réacteur biologique aéré de fabrication de PHA, à savoir, le RAPHA, tandis que la fraction rejetée sera purgée du système, dans son intégralité jusqu'à l'hydrodistillateur, pour l'extraction de PHA à partir de bactéries surproductrices de PHAjusqu'à ce que la concentration de solides dans le RAPHA chute en dessous de 12 g/l, moment à laquelle l'usine UFᵣₐₚₕₐ cessera de fonctionner.

14. Procédé de traitement de matière organique pour la production de PHA et de matière compostable selon la revendication 12, dans lequel l'hydrodistillateur, qui est alimenté avec de ce qui est purgé de l'UFᵣₐₚₕₐ, fonctionne par lots avec les phases suivantes :
a Remplir avec le rejet d'UF_{RAPHA},
b Remuer le mélange,
c Doser la solution basique à partir d'un mélange NaOH/KOH, jusqu'à atteindre une valeur de 11 ≤ pH ≤ 14,
d Chauffer le mélange et soumettre celui-ci à la lyse cellulaire au moyen de la fourniture de chaleur et au moyen de l'utilisation du système à ultrasons, respectivement, jusqu'à ce que les valeurs de consigne de la pression et de la température soient atteintes, maintenir lesdites conditions de pression et de température pendant une durée de 30 à 120 minutes, suffisante pour stériliser le mélange et libérer la PHA contenue dans les bactéries surproductrices de PHA,
e Pressuriser le mélange à la fin de la durée de chauffage pendant 1 min à 15 min,
f Enfin, dépressuriser le mélange,
g Vider sur un filtre sous pression et sous vide.
dans lequel la décharge de liquide stérilisé riche en PHA de l'hydrodistillateur peut être séparée de la fraction liquide à l'issue du filtre.

15. Procédé de traitement de matière organique pour la production de PHA et de matière compostable selon la revendication 13, dans lequel le PHA retenu dans le filtre est lavé avec un flux de KOH/NaOH jusqu'à ce qu'il soit exempt de matière organique, suivi d'un lavage à l'eau d'osmose et enfin d'un séchage par un flux d'air chaud et sec.

16. Procédé de traitement de matière organique pour la production de PHA et de matière compostable selon la revendication 8, dans lequel le biostimulant racinaire est composé de :
a une fraction inorganique riche en potassium et en phosphore et une autre fraction organique riche en azote issues d'acides aminés, issues de la régénération des colonnes d'échange d'ions,
b une fraction de matière organique issue de ce qui est purgé du rejet de l'UF_{AGV} du fermenteur acidogène thermophile générateur d'AGV,
et dans lequel les mélanges des deux fractions sont déshydratés et séchés au moyen d'un séchoir solide à air chaud.
